Europäisches Patentamt

European Patent Office

Office européen des brevets

(19)

(11) Publication number: **0 087 924**
**A2**

(12) **EUROPEAN PATENT APPLICATION**

(21) Application number: **83300965.7**

(22) Date of filing: **24.02.83**

(51) Int. Cl.³: **C 07 C 27/12**

(30) Priority: **01.03.82 FR 8203356**

(43) Date of publication of application:
**07.09.83 Bulletin 83/36**

(84) Designated Contracting States:
**BE DE FR GB IT NL**

(71) Applicant: **The British Petroleum Company p.l.c.**
**Britannic House Moor Lane**
**London EC2Y 9BU(GB)**

(72) Inventor: **Barton, Derek H.R. Bat A/B/C. Centre**
**Residentiel**
**Centre Nat. de la Rech. Sc. Route de Chateaufort**
**F-91190 Gif-sur-Yvette(FR)**

(72) Inventor: **Gastiger, Michel Jacques**
**9 chemin de la Gourdillerie**
**F-91190 Gif-sur-Yvette(FR)**

(72) Inventor: **Motherwell, William Branks**
**3 Residence des Quinconces Route de Chateaufort**
**F-91190 Gif-sur-Yvette(FR)**

(74) Representative: **Harry, John et al,**
**BP INTERNATIONAL LIMITED Patents and Licensing**
**Division Chertsey Road**
**Sunbury-on-Thames Middlesex TW16 7LN(GB)**

(54) Process for the catalytic oxidation of a paraffinic hydrocarbon to an alcohol and either an aldehyde or a ketone.

(57) An alcohol and either an aldehyde or a ketone are produced by oxidising in a liquid medium a paraffinic hydrocarbon in the presence of a soluble iron catalyst, molecular oxygen, an acid and a reducing agent which is a zerovalent metal such as iron in powder form.

EP 0 087 924 A2

Croydon Printing Company Ltd

1

## PROCESS FOR THE CATALYTIC OXIDATION OF A PARAFFINIC
## HYDROCARBON TO AN ALCOHOL AND EITHER AN
## ALDEHYDE OR A KETONE

The present invention relates to a process for the production of an alcohol and either an aldehyde or a ketone by oxidising a paraffinic hydrocarbon in the presence of a soluble iron catalyst, a reducing agent and molecular oxygen.

Since the world's known oil reserves are being depleted at a faster rate than exploration is locating new reserves, there is a growing search for alternative long-term sources of chemicals feedstock. Both coal, of which there are large reserves, and natural gases, eg methane and ethane, which are relatively abundant and are in many cases currently flared off as unwanted by-products of oil production, are receiving increasing attention as potential chemicals feedstock. One approach is to convert them to a mixture of carbon monoxide and hydrogen (synthesis gas) using well known technology and thereafter to convert catalytically the synthesis gas into oxygenated hydrocarbons, such as alcohols, acids and esters. Another approach, is to oxidise paraffinic hydrocarbons directly to alcohols under mild conditions. It is with the latter approach that the present invention is concerned.

Although no chemical systems are known which are selective hydroxylation catalysts under ambient conditions, certain enzymes have been observed to give highly specific hydroxylation reactions yielding terminal alcohols. Such reactions occur in the liver under the influence of the porphyrin enzyme cytochrome P450. The path of this reaction is believed to involve a hydrogen donor ($DoH_2$) so that one oxygen atom from the oxygen molecule is incorporated into the alkane

1

and the other into a molecule of water according to the overall
equation:-

$$RH + O_2 + DoH_2 \longrightarrow ROH + H_2O + Do$$

The first reagent system to mimic this vital biological process was
that of Udenfriend, Clark, Axelrod and Brodie reported in J. Biol.
Chem, 1954, 208, 731.  In this a mixture of ferrous ion with ethylene
diamine tetraacetic acid (EDTA) and ascorbic acid in a phosphate
buffer was used to hydroxylate simple aromatic compounds like
acetanilide.  The addition of hydrogen peroxide increased the rate of
the reaction.  Later workers have modified the conditions drastically,
omitting the EDTA and the ascorbic acid and using hydrogen peroxide
instead of oxygen.  It is thus possible to oxygenate cyclohexane to
cyclohexanol and to convert benzene into phenol.  The Udenfriend
system  has also been further modified by leaving out the EDTA and the
ascorbic acid, but keeping oxygen as the oxidant.  For example Kimura
et al (Chem. Pharm. Bull., 1972, 20, 1883) were able to convert
deoxycholic acid into 15 alpha-hydroxydeoxycholic acid using ferrous
sulphate in a disodium hydrogen phosphate buffer.  Mimoun and Roch
(Tetrahedron 1975, 31, 777) used ferrous chloride and hydrazobenzene
with $^3O_2$ as oxidant for the cyclohexane to cyclohexanol conversion.
Ullrich (Z. Naturforsch, 1969, 24B, 699) used a system composed of
ferrous ion and thiosalicyclic acid for hydroxylation of cyclohexane
and aromatic compounds giving a slightly higher selectivity than
earlier systems.  Seto et al (Tet. Letters, 1980, 2565), in model
experiments designed to demonstrate the importance of -SH or $-S^{\ominus}$
binding to the iron atom in the centre of the porphyrin molecule of
cytochrome P450, previously identified by spectroscopic techniques,
recently oxygenated adamantane selectively at the secondary hydrogen
atoms in a very low yield using (Fe Salen)$_2$O with beta-hydroxy ethyl
mercaptan and oxygen.

    A common feature of many of the prior art systems is the use of a
soluble iron catalyst, a reducing agent in the form of a carboxylic
acid which functions also as a source of protons, and molecular
oxygen.  It has now been found that hydroxylation products, ie
alcohols and either aldehydes or ketones, can be obtained in greater yields by using as the reducing agent a zerovalent metal in combination with an acid.

Accordingly, the present invention provides a process for the production of an alcohol and either an aldehyde or a ketone by oxidising in a liquid medium a paraffinic hydrocarbon in the presence of a soluble iron catalyst, a reducing agent and molecular oxygen characterised in that as the reducing agent there is used a zerovalent metal and in combination therewith there is used an acid.

The paraffinic hydrocarbon may suitably be a linear, branched or cyclic paraffin containing from 1 to 20 carbon atoms. Preferably the paraffin is a linear or branched paraffin containing from 1 to 10 carbon atoms or a mixture thereof. Suitable paraffins include methane, ethane, propane, butane, pentane, hexane, heptane, nonane and decane. Mixtures of paraffins such as those commonly occurring in natural gas reserves may be used if so desired.

The molecular oxygen may be substantially pure oxygen or may be diluted with other gases, such as nitrogen. Preferably oxygen is used in the form of air. Alternatively the oxygen may be generated 'in situ' if so desired.

As the reducing agent there is used a zerovalent metal. Suitable metals include zinc, iron, cobalt and nickel, of which iron is preferred. The zerovalent metal may suitably be added in a high surface area form, such as the particulate form or the multifilament form, preferably in powder form.

In combination with the metal there is used an acid. The acid may be either an inorganic acid or an organic acid, preferably an organic acid. Suitably the inorganic acid may be a mineral acid, such as sulphuric acid or hydrochloric acid. The organic acid may suitably be a carboxylic acid which may be a mono-, di-, tri-, etc, carboxylic acid. The carboxylic acid may suitably be substituted with for example halo groups. Suitable carboxylic acids include formic acid, acetic acid, trichloroacetic acid, trifluoroacetic acid, lactic acid, picolinic acid, oxalic acid, malonic acid, succinic acid, malic acid, diglycolic acid, mucic acid, citric acid and tartaric acid. Preferred carboxylic acids are acetic acid, malonic acid and tartaric acid.

As catalyst there may be used any soluble source of iron. Thus there may be used a compound of iron (11) or iron (111), such as for example ferrous sulphate or ferrous chloride. Alternatively, in a preferred embodiment the iron catalyst is generated 'in situ' by reaction of the acid on zerovalent iron added as reducing agent.

The liquid reaction medium may suitably comprise a single solvent selected from oxygenated hydrocarbons, nitrogenous bases and carboxylic acids or may comprise a compatible mixture of two or more solvents selected from the aforesaid solvents and water. Water may be particularly beneficial in certain circumstances. A suitable oxygenated hydrocarbon is acetone. A suitable carboxylic acid is acetic acid. Suitable nitrogenous bases include pyridine, imidazole and tertiary alkylamines, eg triethylamine. Preferred solvents include pyridine, pyridine/water, pyridine/imidazole and acetone/imidazole.

The molecular oxygen may be substantially pure oxygen or may be diluted with other gases, such as nitrogen. Preferably oxygen is used in the form of air. Alternatively the oxygen may be generated 'in situ' if so desired.

Since the reaction involves both a liquid and a solid phase, a phase transfer agent may be employed. Suitable phase transfer agents are quaternary ammonium salts such as tetraalkylammonium halides. A particularly useful phase transfer agent is tetrabutylammonium bromide.

The number of equivalents of acid employed may be at least equal to and is preferably greater than the number of equivalents of zerovalent metal employed in order to ensure that for each electron there must be a corresponding proton. The amount of liquid medium present is not a critical feature of the invention.

The process may be operated over a moderately wide temperature range. Generally, temperatures in the range from 40 to 100°C will be found suitable, though higher temperatures may be employed if desired. The process may be operated below 40°C, if so desired, by employing an initiator. Suitable initiators include hydrogen sulphide, sulphur and selenium. Preferably the initiator is hydrogen sulphide which may be added as such or generated 'in situ' by the reaction of acid on a suitable sulphide, eg lithium sulphide, sodium sulphide, calcium sulphide and iron sulphide. The pressure may suitably be atmospheric but subatmospheric and superatmospheric pressures may be employed. Superatmospheric pressure is preferred when using gaseous alkanes. In batch reactions, the reaction period may suitably be less than 25 hours and is preferably less than 20 hours.

The reaction may be carried out batchwise or continuously, preferably continuously.

In addition to an alcohol or alcohols, an aldehyde or a ketone is generally formed in the reaction. These too are useful products. Depending upon the nature of the paraffinic hydrocarbon reactant, primary, secondary, and tertiary alcohols may be formed. The relative proportions of the various components in the product will depend upon a number of factors, including the reaction conditions, the nature of the solvent and the nature and quantity of the acid employed.

The invention will now be illustrated by reference to the following Examples.

A. **Use of inorganic acid**

Examples 1 to 4

To pyridine (30cm$^3$) in a reaction vessel was added sodium sulphide nonahydrate (0.5g;2mM), adamantane (a paraffinic hydrocarbon) (280mg;2.1mM), iron powder (1g;18mM) and either sulphuric acid or hydrochloric acid. The whole was mixed in an atmosphere of oxygen, usually over a period of 18 hours, at ambient temperature.

At the end of this period, in order to recover unreacted adamantane and oxidation products the liquid phase, ie solution, was then extracted with distilled pentane and washed successively with water and a saturated solution of copper sulphate or HCl (10%) and finally with satuated sodium chloride and dried (MgSO$_4$). The organic phase was concentrated to dryness in a Rotoevaporator. A portion of this residue was injected into a GPC. The GPC column was of the SE30 type, 5% on WAW DMCS, length 1m40. Using a temperature programme of 70°C to 110°C (2°C/min) the retention times of the reaction products were determined: adamantane 350 sec., adamantanol-1 730 sec., adamantanone 870 sec., and adamantanol-2, 960 sec (internal standard : alpha tetralone and/or naphthalene).

The nature of the acid employed, the relative proportions of the reactants, the reaction periods and the results of the GPC analyses are given in Table 1.

B   Use of organic acid

Examples 5 to 22

Iron powder (10 atom equiv) and the organic acid (10 mmol) were added to a solution of adamantane (1 mmol) in pyridine containing 6.6% water (10 ml). Hydrogen sulphide (0.05 mmol) in pyridine was added and a slow stream of oxygen was passed over the vigorously stirred reaction mixture which was maintained in a bath at 30°C.

Thereafter the unreacted adamantane and oxidation products were recovered and analysed by Gas Liquid Chromatography (G.L.C.).

The nature of the organic acid employed, the reaction periods and the results of the G.L.C. analysis are given in Table 2.

C.   Oxidation of cyclohexane

Examples 23 to 38

To a solution of pyridine (28 ml) and distilled water (2 ml) were added varying quantities [between 1.9 mM (0.21 ml) and 46.4 mM (5 ml)] of cyclohexane and of iron powder [0.21 mg (3.6 mM) to 5.2g (92.8 mM)] and acetic acid [0.43 ml (7.6 mM) to 10.6 ml (186 mM)].

This reaction mixture was magnetically stirred in a reaction vessel to which was attached a balloon of oxygen. The treatment was carried out as follows: distilled water (20 ml) was added to the reaction mixture and it was extracted six times with diethyl ether (30 ml). The organic phase was washed with a solution of HCl (10%) and then with a saturated sodium chloride solution. The resulting organic phase was dried with $MgSO_4$, then concentrated by Rotoevaporator. A portion was analysed by GPC [4m OV17 5% from 120 to 160°C (2°C/min):internal standard = toluene].

The conditions and results are given in Table 3.

D. Oxidation of cyclododecane

Example 39

The procedure of Examples 23 to 38 was repeated except that instead of using a balloon of oxygen a stream of oxygen was bubbled through the reaction solution. The details were as follows:

| | |
|---|---|
| Cyclododecane | 6.14 mol |
| Fe | 10 eq |
| AcOH | 20 eq |
| Liquid medium | pyridine/water |
| Temperature | 40°C |
| Reaction time | 48 h |
| Fe(0) recovered | 18% |

Results

| | |
|---|---|
| Cyclododecane | 39% |
| Cyclododecanone | 31% (1.9 mol) |
| Cyclododecanol | 2.6% |

2,4-dinitrophenyl hydrazone derivative of -one:28% (1.74 mM).

E. Variation of quantity of acetic acid

Examples 40 to 47

The procedure employed in Examples 1 to 4 was followed except that acetic acid was used in place of the mineral acid. The quantity of acetic acid was varied within the range from 1.9 mM to 100%, sodium sulphide from 0.2mM to 11mM and iron from 2mM to 20mM.

The relative proportions of the reactants, the reaction periods and the results of the GPC analysis are given in Table 4.

F. Variation of the solvent

Examples 48 to 53

The procedure employed in Examples 1 to 4 was repeated and in place of pyridine a variety of solvents was employed.

The amounts of the various reactants, the nature of the solvent and the results of the GPC analysis are given in Table 5.

G. Variation of the temperature

Examples 54 to 57

The procedure of Examples 1 to 4 was repeated at temperatures in the range 0 to 80°C.

The amounts of reactants, the reaction conditions (time and temperature) and the results of the GPC Analysis of the products are given in Table 6.

H. Variation of the amount of sodium sulphide and iron

Examples 58 to 63

The procedure of Examples 1 to 4 was repeated using different quantities of sodium sulphide and iron.

The amounts of reactants, reaction time and results of the GPC analysis of the products are given in Table 7.

I. Use of hydrogen sulphide supplied from external source

Examples 64 to 69

Into pyridine (distilled over potassium hydroxide and stored on 4A molecular sieves) was bubbled gaseous hydrogen sulphide (20 mM). The hydrogen sulphide concentration of the resulting solution was 0.4g/50ml pyridine.

To 30 cm$^3$ of pyridine and adamantane was added 0.03g (1mM) of hydrogen sulphide in the form of the solution prepared as above and 1.07 g (19mM) of iron powder. The resulting mixture was left under an atmosphere of oxygen for 18 hours and thereafter the mixture was treated in the manner described for Examples 1 to 4.

The amounts of reactants, the reaction conditions and the results of the GPC analysis are given in Table 8.

J. Use of a phase transfer agent

Examples 70 to 72

The procedure of Examples 1 to 4 was employed except that a phase transfer agent, tetrabutylammonium bromide, was added to the reaction mixture.

The quantities of reactants, the reaction times and the results of GPC analysis of the products are given in Table 9.

K. Variation of the cation and improvements attending the addition of water

Examples 73 to 82

The procedure employed in Examples 1 to 4 was followed except that instead of sodium sulphide a variety of metal sulphides (1 mol eq.) was employed. Acetic acid (10 mol eq.) and iron powder (10 atom eq.) were used, except in Examples 49 and 50 in which ferrous sulphide in the form of iron pyrites was added (in place of iron powder). In Examples 51, thiourea (1 mol eq.) was used in place of sodium sulphide. In Examples 74, 76, 78, 81 and 82 a pyridine-water (6.6%) mixture was used in place of pyridine alone.

The nature of the sulphide and the results of the GPC analysis are given in Table 10.

0087924

Table 1
Use of inorganic acid

| Ex. | Adamantane (mol equivs) | Acid (mol equivs) | Na$_2$S·9H$_2$O (mol equivs) | Fe (atom equivs) | Reaction time (hours) | Solvent | Products | | |
|---|---|---|---|---|---|---|---|---|---|
| | | | | | | | adamant- anol-1 | adamant- anone | adamant- anol-2 |
| 1 | 1 | H$_2$SO$_4$ (2.1) | 1 | 10 | 19 | Pyridine | 0.34 | | 1.7* |
| 2 | 1 | H$_2$SO$_4$ (10) | 1 | 10 | 6 | " | 0.07 | | 0.5* |
| 3 | 1 | H$_2$SO$_4$ (4) | 1 | 10 | 18 | " | 0.3 | | 1.7* |
| 4 | 1 | HCl (3.6) | 1 | 10 | 19 | " | 0.07 | | 0.5* |

*adamantanone + adamantanol-2

## Table 2

### Use of organic acid

| Example | Acid | Time (h) | PRODUCTS (% yield) | | | Total oxidation (% yield) | $c^2/c^3$ [a] |
|---|---|---|---|---|---|---|---|
| | | | Adamantan-1-ol | Adamantan-2-ol | Adamantanone | | |
| 5 | Acetic | 15.5 | 3.7 | – | 9.4 | 13.1 | 2.54 |
| 6 | Trichloroacetic | 16.5 | 2.7 | – | 5.95 | 8.65 | 2.2 |
| 7 | Trifluoroacetic | 17.5 | 1.7 | – | 9.0 | 10.7 | 5.3 |
| 8 | d(+) Lactic | 16 | 2.8 | – | 4.8 | 7.6 | 1.7 |
| 9 | Picolinic | 17 | 3.6 | – | 7.04 | 10.6 | 1.96 |
| 10 | Thiosalicylic | 15 | 1.8 | 0.2 | 1.2 | 3.2 | 0.8 |
| 11 | Oxalic | 21.5 | 1.4 | – | 4.3 | 5.7 | 3.07 |
| 12 | Malonic | 19.5 | 4.0 | – | 15.3 | 19.3 | 3.8 |
| 13 | Succinic | 16 | 3.4 | – | 4.7 | 8.1 | 1.4 |
| 14 | dl Malic | 19 | 1.0 | – | 3.04 | 4.04 | 3.0 |
| 15 | Diglycolic | 17 | 3.4 | – | 12.02 | 15.4 | 3.5 |
| 16 | Mucic | 27.5 | 8.02 | – | 12.3 | 20.3 | 1.5 |
| 17 | Citric | 15 | 6.2 | – | 15.4 | 21.6 | 2.5 |
| 18 | meso Tartaric | 17.5 | 8.6 | – | 16.6 | 25.2 | 1.9 |
| 19 | d(+) Tartaric | 15 | 9.2 | – | 17.5 [b] | 26.7 | 1.9 |
| 20 | d(+) Tartaric | 2 | 6.9 | – | 14.7 | 21.6 | 2.1 |
| 21 | d(+) Tartaric (c) | 3 | 1.6 | – | 14.3 | 15.9 | 9.0 |
| 22 | d(+) Tartaric (d) | 49 | 1.6 | – | 13.7 | 15.3 | 8.6 |

(a) $c^2/c^3$ represents the relative ratio of attack at the secondary and tertiary positions respectively.

(b) the crystalline 2,4-dinitrophenylhydrazone derivative of adamantanone was isolated in 18.6% yield.

(c) reaction performed under a static oxygen atmosphere.

(d)      "          "          "   "   "    "              "          using 20 ml of solvent.

## Table 3

### Use of cyclohexane

| Example | Cyclohexane (mM) | Fe (eq) | AcOH (eq) | Reaction time (h) | Fe(0) recovered (%) | PRODUCTS (% yield) | |
|---|---|---|---|---|---|---|---|
| | | | | | | Cyclohexanol | Cyclohexanone |
| 23 | 1.9 | 30 | 60 | 22 | 13 | 2.1 | 5 |
| 24 | 1.9 | 30 | 60 | 5 | 59 | 1.4 | 5.9 |
| 25 | 5.6 | 10 | 20 | 21 | 15 | 1.8 | 7.4 |
| 26 | 9.3 | 2 | 4 | 23 | 5 | 0.55 | 6.4 |
| 27 | 9.3 | 4 | 8 | 47 | 7 | 0.8 | 2.1 |
| 28 | 9.3 | 10 | 20 | 22 | 54 | 1.1 | 3.5 |
| 29 | 13.9 | 4 | 8 | 23 + | 16 | 1.6 | 16.5 |
| 30 | 18.6 | 5 | 10 | 36 | 39 | 1.1 | 2.8 |
| 31 | 27.9 | 2 | 4 | 23 | 0 | 2 | 11 |
| 32 | 27.9 | 2 | 4 | 22 | 15 | 1.5 | 4.7 |
| 33 | 27.9 | 2 | 4 | 53 | 0 | 1.4 | 5.7** |
| 34 | 27.9 | 2 | 4 | 17 + | 21 | 1 | 10.5** |
| 35 | 27.9 | 5 | 10 | 24 | 58 | 0.9 | 2.5 |
| 36 | 27.9 | 2 | 2* | 45 | 41 | 0.1 | 1.9 |
| 37 | 46.4 | 1.2 | 2.4 | 15 | 11 | 1.1 | 3.6** |
| 38 | 46.4 | 2 | 4 | 23 | 66 | 0.18 | 1.15 |

* with tartaric acid

** by dinitrophenylhydrazone

+ flux oxygen ie oxygen bubbled through reaction solution

Table 4

Variation in quantity of acetic acid

| Example | Acetic acid (mol equ.) | Na$_2$S.9H$_2$O (mol equivs) | Fe (atom equivs) | Time (hours) | Products | | |
|---|---|---|---|---|---|---|---|
| | | | | | adamantanol-1 | adamantanone | adamantanol-2 |
| 40 | 1 | 1 | 10 | 18 | 0.21 | 0.30 | 0.26 |
| 41 | 5 | 1 | 10 | 18 | 0.28 | 0.90 | 0.43 |
| 42 | 10 | 1 | 10 | 118 | 0.9 | 3.7 | 0.2 |
| 43 | 10 | 1 | 10 | 22 | 0.5 | 3.2 | 0.4 |
| 44 | 10% | 1 | 10 | 18 | 0.52 | 1.4 | 1.2 |
| 45 | 20% | 1 | 10 | 18 | 0.21 | 0.65 | 0.25 |
| 46 | 50% | 1 | 10 | 22 | 0.28 | 2.7 | 0.7 |
| 47 | 100% | 1 | 10 | 18 | 1.3 | 0.6 | 2.7 |

Table 5

Variation of solvent

| Example | Adamant -ane mol eq | Acetic acid (mol eq) | Na$_2$S.9H$_2$O (mol eq) | Solvent | Fe (atom equivs) | Products | | |
|---|---|---|---|---|---|---|---|---|
| | | | | | | adamantanol-1 | adamantanone | adamantanol-2 |
| 48 | 1 | 10 | 1 | Acetone | 10 | 0.3 | 1.05* | |
| 49 | 1 | 10 | 1 | Acetone/pyridine(3%) | 10 | 0.12 | 2.4* | |
| 50 | 1 | 10 | 1 | Pyridine/water (3%) | 10 | 0.27 | 2 | 0.7 |
| 51 | 1 | 10 | 1 | Acetone/Et$_3$N (3%) | 10 | 0.29 | 0.28 | 0.36 |
| 52 | 1 | 10 | 1 | Acetone/imidazole (3%) | 10 | 0.40 | 0.62 | 0.70 |
| 53 | 1 | 10 | 1 | Pyridine/imidazole(3%) | 10 | 0.33 | 2.23 | 0.37 |

Reaction Time = 18 hours

* adamantanone + adamantanol

## Table 6

### Variation of temperature

| Example | adamantane (mol equivs) | acetic acid (mol equivs) | Fe (mol equivs) | $Na_2S.9H_2O$ (equivs) | Time(hours) and Temperature (°C) | Solvent | Products | | |
|---------|-------------------------|--------------------------|-----------------|------------------------|----------------------------------|---------|------------------|----------------|----------------|
| | | | | | | | adamantanol-1 | adamantanone | adamantanol-2 |
| 54 | 1 | 10 | 10 | 1 | 18 hours 80°C | Pyridine | 0.19 | 0.60 | 1.26 |
| 55 | 1 | 10 | 10 | 1 | 18 hours 40°C | Pyridine | 0.23 | 1.0 | 0.74 |
| 56 | 1 | 10 | 10 | 1 | 7½ hours 0°C | Pyridine | 0.36 | 0.86 | 0.41 |
| 57 | 1 | 10 | 10 | 1 | 7½ hours ambient | Pyridine | 0.50 | 3.0 | 0.58 |

Table 7

Variation of the amounts of sodium sulphide and iron

| Example | adamantane (mol eqivs) | acetic acid (mol eq) | Fe (atom equivs) | $Na_2S.9H_2O$ (mol equivs) | Time (hours | Products | | |
|---------|---------|---------|---------|---------|---------|---------|---------|---------|
| | | | | | | adamantanol-1 | adamantanone | adamanotanol-2 |
| 58 | 1 | 100% | 10 | 6 | 50 | 2.3 | 1.60 | 2.2 |
| 59 | 1 | 10 | 10 | 6 | 50 | 0.6 | 3.1 | 0.7 |
| 60 | 1 | 12 | 10 | 6 | 50 | 0.28 | 1.6* | |
| 61 | 1 | 10 | 10 | 0.5 | 18 | 0.54 | 1.9 | 0.3 |
| 62 | 1 | 10 | 10 | 0.1 | 18 | 0.15 | 0.3 | less than 0.01 |
| 63 | 1 | 10 | 1 | 1 | 18 | 0.17 | 0.9 | 0.10 |

Table 8

Use of externally generated hydrogen sulphide

| Example | Adamantane (mol equivs) | H$_2$S (mol equivs) | Fe (atom equivs) | Time (hours) | Solvent | Products | | |
|---|---|---|---|---|---|---|---|---|
| | | | | | | adamantanol-1 | adamantanone | adamantanol-2 |
| 64 | 1 | 0.5 | 10 | 18 | pyridine | 0.04 | 0.21 | 0.05 |
| 65 | 1 | 0.5 | 10 | 18 | pyridine/Et$_3$N(3%) | 0.02 | 0.03 | 0.04 |
| 66 | 1 | 2 | 10 | 7 | pyridine | - | - | - |
| 67 | 1 | 2 | 10 | 18 | " | less than 0.01 | 0.7* | |
| 68 | 1 | 0.24 | 10 | 118 | " | 0.21 | 0.8* | |
| 69 | 1 | 8.1 | 10 | 18 | " | 0.02 | 0.18 | 0.08 |

* adamantanone + adamantanol-2

Table 9

Use of a phase transfer agent

| Example | Adamantane (mol equivs) | Na$_2$S.9H$_2$O (mol equivs) | Fe (atom equivs) | acetic acid (mol eq) | Bu$_4$NBr (%) | Time (hours) | Products | | |
|---------|---------|---------|---------|---------|---------|---------|---------|---------|---------|
| | | | | | | | adamantanol-1 | adamantanone | adamantanol-2 |
| 70 | 1 | 1 | 10 | 10 | 10 | 18 | 0.66 | 2.5 | 1.2 |
| 71 | 1 | 1 | 10 | 10 | 11 | 18 | 0.47 | 1.5 | 0.4 |
| 72 | 1 | 1 | 10 | 100% | 9 | 18 | 2.9 | 0.5 | 3.5 |

Table 10

| Ex. | Metal Sulphide (mol. equiv.) | | Solvent | Products | | |
|---|---|---|---|---|---|---|
| | | | | adamantanol-1 | adamantanone | adamantanol-2 |
| 73 | $K_2S$ | (1.0) | Pyridine | 0.13 | 0.30 | |
| 74 | $K_2S$ | (1.0) | Pyridine-$H_2O$ (6.6%) | 1.1 | 5.2 | 0.9 |
| 75 | $Li_2S$ | (1.0) | Pyridine | 0.10 | 0.40 | |
| 76 | $Li_2S$ | (1.0) | Pyridine-$H_2O$ (6.6%) | 1.1 | 4.2 | 0.6 |
| 77 | CaS | (1.0) | Pyridine | 0.4 | 0.75 | |
| 78 | CaS | (1.0) | Pyridine-$H_2O$ (6.6%) | 1.1 | 7.2 | |
| 79 | $(NH_4)_2S$ | (1.0) | Pyridine-$H_2O$ | 0.1 | 0.4 | |
| 80 | # $Fe^{ii}S$ | (1.0) | Pyridine | 0.8 | 1.3 | |
| 81 | # $Fe^{ii}S$ | (1.0) | Pyridine-$H_2O$ (6.6%) | 1.5 | 3.0 | |
| 82 | Thiourea | (1.0) | Pyridine-$H_2O$ (6.6%) | 2.0 | 3.0 | 1.7 |

All reactions were run for 18 hours at room temperature with 10 eq. HOAc and 10 eq. Fe powder unless otherwise stated.

# Fe powder was not added $Fe^{ii}S$ = iron pyrites.

Claims:

1. A process for the production of an alcohol and either an aldehyde or a ketone by oxidising in a liquid medium a paraffinic hydrocarbon in the presence of a soluble iron catalyst, a reducing agent and molecular oxygen

characterised in that

as the reducing agent there is used a zerovalent metal and in combination therewith there is used an acid.

2. A process according to claim 1 wherein the paraffinic hydrocarbon is a linear or branched paraffin containing form 1 to 10 carbon atoms or a mixture thereof.

3. A process according to either claim 1 or claim 2 wherein the molecular oxygen is supplied in the form of air.

4. A process according to any one of claims 1 to 3 wherein the zerovalent metal is either zinc, iron, cobalt or nickel.

5. A process according to any one of claims 1 to 4 wherein the acid is a carboxylic acid.

6. A process according to claim 5 wherein the carboxylic acid is either acetic acid, malonic acid or tartaric acid.

7. A process according to any one of the previous claims wherein the zerovalent metal is iron and the soluble iron catalyst is generated 'in situ' by reaction with acid.

8. A process according to any one of the previous claims wherein the liquid medium comprises either pyridine, pyridine/water, pyridine/imidazole or acetone/imidazole.

9. A process according to any one of the preceding claims wherein the oxidation is carried out at a temperature in the range 40 to 100°C.

10. A process according to any one of claims 1 to 9 wherein the oxidation is carried out at a temperature below 40°C, employing as an initiator either hydrogen sulphide, sulphur or selenium.